# EUROPEAN PATENT SPECIFICATION

(11) **EP 4 561 469 B1**
(45) Date of publication and mention of the grant of the patent: **22.07.2026**
(21) Application number: 23806409.1
(22) Date of filing: 12.10.2023
(51) Int. Cl.: A61B 17/34, A61B 17/00, A61B 90/00

(54) **VARIABLE EXPOSURE NEEDLE**
NADEL MIT VARIABLER EXPOSITION
AIGUILLE D'EXPOSITION VARIABLE

(30) Priority: 07.12.2022 US 202263386415 P
(43) Date of publication of application: 04.06.2025
(73) Proprietor: Boston Scientific Scimed, Inc., Maple Grove, MN 55311 (US)
(72) Inventor: THOMSON, Perry, Somerville, Massachusetts 02144 (US); GAVALIS, Robb Morse, Westborough, Massachusetts 01581 (US); ROMANY, Cristina, Watertown, Massachusetts 02472 (US)
(74) Representative: Vossius & Partner Patentanwälte Rechtsanwälte mbB
(86) International application number: PCT/US2023/035042
(87) International publication number: WO 2024/123414

(56) References cited:
- WO-A1-2015/088961
- WO-A1-2022/022169

## Description

### Filed

The present disclosure relates to a variable exposure needle for Endoscopic Ultrasound (EUS) procedures.

### Background

Endoscopic Ultrasound (EUS) is a minimally invasive procedure performed with a specialized endoscope that uses high frequency soundwaves to visualize, for example, the digestive (gastrointestinal) tract and other nearby structures. According to one application, EUS is used to facilitate direct biliary drainage when, for example, a traditional endoscopic retrograde cholangiopancreatology (ERCP), which utilizes contrast dye and X-rays to identify and treat blockages within the ducts via the papilla, has failed. EUS may be used to visualize the ducts directly through the gastric wall, facilitating puncture with a needle to gain guidewire access to the common bile or pancreatic ducts.

Successful EUS access procedures generally depend on initial duct puncture and access cannula stability within the duct while passing a guidewire to the target site. However, the ability to easily puncture the duct while also maintaining stability for guidewire access may, in some cases, be difficult to achieve. In addition, the use of needles to puncture ducts, particularly smaller ducts, poses an inherent risk for a through and through puncture in which the needle tip not only pierces the near wall of the duct, but passes through a far wall of the duct as well.

WO 2015/088961 A1 discloses a stylet for use with a minimally invasive medical instrument, comprising: an elongate, flexible member extending from a proximal end to a distal end and including a sensor system that is substantially aligned with at least a portion of the elongate member, the sensor system being configured to measure characteristics of the elongate, flexible member, wherein the elongate, flexible member is sized and shaped to be slidably received within a lumen of the medical instrument.

WO 2022/022169 A1 discloses an endoscopic surgical instrument, comprising a needle, an actuator and an outer tube. The needle can extend out of the outer tube and retract into the outer tube under the actuation action of the actuator. The endoscopic surgical instrument further comprises a protective device for preventing the outer tube from being punctured by the needle when bent, and the protective device comprises a protective tube disposed on an inner wall of the outer tube. When the needle is retracted into the outer tube, the protective tube at least surrounds the needle tip of the needle.

### Summary

The present invention relates to a system for accessing a biliary duct as defined in independent claim 1. The system includes an access cannula extending along a longitudinal axis from a proximal end to a distal end and including a channel extending longitudinally therethrough. A distal portion of the access cannula includes a laser cut pattern including a plurality of slots extending through a wall thereof so that the access cannula is movable between a biased non-compressed configuration and a compressed configuration, in which the access cannula is compressed along the longitudinal axis.

The system also includes a needle extending longitudinally from a proximal end to a sharp distal end. The needle is housed within the channel of the access cannula so that, when the access cannula is in the non-compressed configuration, the sharp distal end is covered via the access cannula and, when the access cannula is in the compressed configuration, the sharp distal end is exposed to puncture a wall of one of an organ and a target duct.

In an embodiment, the laser cut pattern includes a plurality of rows, each row extending about a periphery of the access cannula, each of the rows including at least one slot extending along a portion of the row, through the wall such that the channel of the access cannula is open to and in communication with an exterior of the access cannula via the slots.

In an embodiment, each row of the laser cut pattern extends perpendicular to the longitudinal axis of the access cannula.

In an embodiment, the laser cut pattern is selected to achieve a desired level of compressibility for the access cannula, the desired level of compressibility including a compressive force required to move the access cannula from the non-compressed configuration toward the compressed configuration and length by which the access cannula is shortened when moved from the non-compressed configuration toward the compressed configuration.

In an embodiment, the desired level of compressibility achieved via the laser cut pattern is based on one of a number of slots per row, dimensions of each of the slots, a rotation angle between corresponding slots in adjacent rows, and a distance between rows.

In an embodiment, in the non-compressed configuration, a distal-most tip of the needle protrudes distally of the distal end of the access cannula.

In an embodiment, when the access cannula is moved from the non-compressed configuration to the compressed configuration, the access cannula is shortened via a distance corresponding to the sharp distal end of the needle.

In an embodiment, a length of the sharp distal end of the needle that is exposed when the access cannula is in the compressed configuration is smaller than the target duct such to prevent a through and through puncture.

In an embodiment, the access cannula is formed of a nitinol hypotube.

The present disclosure also relates to a needle system for treating a biliary duct. The system includes an access cannula sized, shaped and configured to be inserted through a working channel of an endoscope. The access cannula extends along a longitudinal axis from a proximal end to a distal end and has a substantially tubular body. A portion of the access cannula including a laser cut pattern including a plurality of slots extending through a wall of the access cannula so that the access cannula is compressible along the longitudinal axis thereof.

The system includes a needle removably received within the access cannula and extending longitudinally from a proximal end to a sharp distal end. The sharp distal end is covered via the access cannula when in the access cannula is in non-compressed configuration and exposed when the access cannula is in a compressed configuration.

In an embodiment, the access cannula is biased toward the non-compressed configuration so that the access cannula is movable between the non-compressed and compressed configurations.

In an embodiment, the laser cut pattern includes a plurality of rows, each row extending about a periphery of the access cannula, each of the rows including at least one slot extending along a portion of the row, through the wall such that the channel of the access cannula is open to and in communication with an exterior of the access cannula via the slots.

In an embodiment, the laser cut pattern is selected to achieve a desired level of compressibility for the access cannula, the desired level of compressibility including a compressive force required to move the access cannula from the non-compressed configuration toward the compressed configuration and length by which the access cannula is shortened when moved from the non-compressed configuration toward the compressed configuration.

In an embodiment, the desired level of compressibility achieved via the laser cut pattern is based on (a) a number of slots per row, (b) dimensions of each of the slots, (c) a rotation angle between corresponding slots in adjacent rows, and/or (d) a distance between rows.

In an embodiment, the access cannula is formed of a nitinol hypotube.

The present disclosure describes a method for accessing a biliary duct. No methods of surgery or treatment are claimed. The method includes inserting an endoscope to target area within a stomach; inserting an access cannula, in a non-compressed configuration, through a working channel of the endoscope and positioning a distal end of the access cannula against a portion of a wall of the stomach adjacent a target wall of a target duct to be accessed, wherein the access cannula includes a laser cut pattern including a plurality of slots extending through a wall thereof so that the access cannula is movable between the non-compressed configuration, in which a sharp distal end of a needle housed therewithin is covered, and a compressed configuration; pressing the access cannula distally against the wall of the stomach until the access cannula is moved from the non-compressed configuration toward the compressed configuration, in which the access cannula is compressed along a longitudinal axis thereof to expose the sharp distal end of the needle such that the sharp distal end punctures the portion of the wall of the stomach over which the access cannula was positioned; and moving the access cannula further distally relative to the endoscope so that the sharp distal end of the needle punctures the target wall of the target duct, the access cannula reverting toward the non-compressed configuration upon puncturing of the target wall of the target duct to extend over the sharp distal end of the needle into the target duct.

In an embodiment, a compressive force required to maintain the access cannula toward the compressed configuration exceeds the compressive force provided via the punctured target wall such that, the access cannula reverts toward the non-compressed configuration upon puncturing of the target wall of the target duct to prevent the needle from damaging a far wall of the target duct.

In an embodiment, the reversion of the access cannula toward the non-compressed configuration increases a length of the access cannula within the target duct to secure the access cannula therein.

In an embodiment, the method further includes removing the needle from within the access cannula; and inserting a guidewire through the access cannula such that the guidewires is inserted into the target duct.

In an embodiment, the laser cut pattern is selected to achieve a desired level of compressibility for the access cannula, the desired level of compressibility including a compressive force required to move the access cannula from the non-compressed configuration toward the compressed configuration and length by which the access cannula is shortened when moved from the non-compressed configuration toward the compressed configuration to prevent a through and through puncture of the target duct.

### Brief Description

Fig. 1 shows a longitudinal side view of a distal portion of a system according to an exemplary embodiment of the present disclosure, in a non-compressed configuration;
Fig. 2 shows a longitudinal side view of a distal portion of the system according to Fig. 1, in a compressed configuration;
Fig. 3 shows a cross-sectional view of the system according to Fig. 1, along line X;
Fig. 4 shows an enlarged longitudinal side view of the system according to Fig. 1, including a first exemplary laser cut pattern:
Fig. 5 shows an enlarged longitudinal side view of the system according to Fig. 1, including a second exemplary laser cut pattern;
Fig. 6 shows an enlarged longitudinal side view of the system according to Fig. 1, including a third exemplary laser cut pattern;
Fig. 7 shows a longitudinal side view of a distal portion of the system according to Fig. 1, an access cannula thereof positioned against a wall of an organ, in the non-compressed configuration:
Fig. 8 shows a longitudinal side view of a distal portion of the system according to Fig. 1, the access cannula in the compressed configuration so that the sharp end of a needle is exposed to puncture the wall of the organ;
Fig. 9 shows a longitudinal side view of a distal portion of the system according to Fig. 1, the sharp end of the needle puncturing a target wall of a target duct; and
Fig. 10 shows a longitudinal side view of a distal portion of the system according to Fig. 1, the access cannula reverting toward the non-compressed configuration to move distally over the sharp end of the needle and access the target duct.

### Detailed Description

The present disclosure may be further understood with reference to the following description and appended drawings, wherein like elements are referred to with the same reference numerals. The present disclosure relates to an endoscopic needle system and, in particular, relates to a variable exposure needle system for EUS procedures. Exemplary embodiments of the present disclosure comprise a system including an access cannula configured to be longitudinally compressed to expose a sharp end of a needle housed therein. The access cannulas of these embodiments include a cut pattern (e.g., laser cut) extending along a portion thereof to permit a desired level of longitudinal compression and recoil of the access cannula so that, when a distal end of the access cannula is pressed against a wall of an organ and/or a biliary duct, the access cannula is compressed to expose only a desired portion of the sharp end of the needle so that the user may puncture the organ and/or duct wall against which the access cannula is pressed without also puncturing a far wall of the organ/duct.

It should be noted that although the exemplary embodiments specifically describe a needle system for accessing a biliary duct for, for example, a biliary drainage procedure, it will be understood by those of skill in the art that the exemplary system may be utilized for any of variety procedures in which a compressive access cannula, a controlled puncture distance, or exposed sharp only when in contact of tissue may be desired. It should also be noted that the terms "proximal" and "distal," as used herein, are intended to refer to a direction toward (proximal) and away from (distal) a user of the device (e.g., physician).

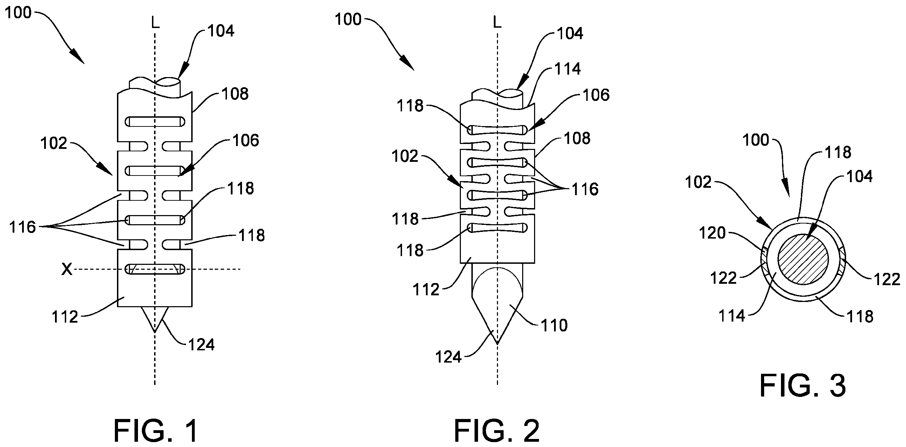

As shown in Figs. 1-10, a needle access system 100 for treating, for example, a biliary duct, according to an exemplary embodiment of the present disclosure comprises an access cannula 102 and a needle 104 housed therein. The access cannula 102 includes a pattern 106 of openings extending (e.g., a laser cut pattern) along a distal portion 108 of the access cannula 102. The pattern 106 is configured to facilitate longitudinal compression of the access cannula 102 so that the access cannula 102 is movable between a non-compressed configuration (as shown in Fig. 1), in which a sharp end 110 of the needle 104 is housed within the distal portion 108, and a compressed configuration (as shown in Fig. 2), in which a length of the access cannula 102 is shortened so that a selected length of the distal portion of the needle 104 including the sharp end 110 is exposed to puncture a wall of, for example, an organ and/or a target duct. In an exemplary embodiment, the system 100 is inserted to a target area within a patient body via, for example, a working channel of an endoscope that has been previously placed.

The system 100 is inserted to the target area with the access cannula 102 in the non-compressed configuration and positioned adjacent to a portion of tissue to be punctured (an organ wall such as, for example, the stomach) to obtain access to the target duct. The access cannula 102 along with the needle 104 are pressed distally against the wall of the organ to compress the distal portion 108 of the access cannula 102 against the wall of the organ. This moves the distal portion 108 of the access cannula 102 from the non-compressed configuration to the compressed configuration, thereby exposing a desired length of the sharp end 110.

The user may then push the exposed sharp end 110 distally relative to the endoscope (along with the access cannula 102) to puncture the wall of the organ. When the sharp end 110 of the needle 104 has punctured the wall of the organ, the access cannula 102 will be moved further distally to also pass through the organ wall and pierce a near (target) wall of the target duct. As will be described in further detail below, the system 100 is configured so that, when the exposed sharp end 110 passes into the target duct and the compressive force from the tissue is removed from the distal portion 108, the distal portion 108 expands from the compressed configuration toward the non-compressed configuration. That is, when the sharp end 110 of the needle 104 enters the duct, the cannula 102 expands to cover the distal portion of the needle 104 to prevent the sharp end 110 from also puncturing a far wall of the target duct. Once the target wall of the duct has been punctured, a compressive force exerted on the access cannula 102 from the passing tissue is reduced so that the access cannula 102 reverts toward the non-compressed configuration, moving distally at least partially over the sharp end 110 and into the target duct.

Those skilled in the art will understand that the degree of reduction in the compressive force exerted on the distal portion 108 of the access cannula 102 will vary depending on various factors such as the qualities (e.g., density) of the tissue through which the cannula 102 has been passed, differences between tissues/disease states, the length of the distal portion 108 that remains within the tissue when the distal end of the cannula 102 enters the target duct, etc. so that the sharp end 110 of the needle 104 may not be entirely covered by the reversion of the cannula 102 toward the non-compressed configuration. However, by shortening the length of the needle 104 that is exposed from the distal end of the cannula 102, the potential to penetrate/damage the far wall of the target duct is significantly reduced. The needle 104 may then be removed from the access cannula 102 so that other devices and/or tools such as, for example, a guidewire, may be inserted into the target duct via the access cannula 102.

The access cannula 102 extends along a longitudinal axis L from a proximal end (not shown) to a distal end 112 and includes a channel 114 extending therethrough. As described above, the distal portion 108 of the access cannula 102 includes a pattern 106 of openings extending therealong that facilitates movement of the access cannula 102 between the non-compressed configuration, toward which the access cannula 102 is biased, and the compressed configuration. As would be understood by those skilled in the art, the pattern 106 (e.g., a size and spacing of the openings in the cannula 102 as related to a diameter of the cannula 102) may be selected to achieve a desired level of compressibility (i.e., a length by which the cannula 102 will be compressed when subject to a level of force associated with penetration of the tissue to be penetrated).

In an exemplary embodiment, the level of compressibility may be selected so that the access cannula 102, when moved from the non-compressed to the compressed configuration, may be shortened via a distance selected to expose a desired length of the needle 104 (e.g., the sharp end 110) based on, for example, a size of a target duct to be accessed. In particular, a length of the exposed sharp end 110 should be such that, after the sharp end 110 pierces a near wall of the target duct, the distal end of the cannula 102 enters the target duct and the distal portion 108 recoils to cover the sharp end 110 before the sharp end 110 punctures a far wall of the target duct.

In an exemplary embodiment, the pattern 106, as will be described in further detail below, may be selected to control a force required to compress the access cannula 102 an amount (i.e., length) of compression by which the access cannula is reduced. The compressive force may be tuned so that it is slightly less than an anatomical tissue of the target duct/structure so that access cannula 102 is permitted to compress and expose the sharp end 110 of the needle 104. The compressive length is tuned so that it corresponds to or is slightly less than the target duct/structure so as not to damage the far wall thereof as the needle 104 is inserted thereinto. This may involve, in one embodiment, engineering spring forces or discrete compressive zones with different rates or hard stops. In another embodiment, pattern 106 may generate a variable compressive rate between the non-compressed (e.g., relaxed) and compressed configurations.

In an exemplary embodiment, the pattern 106 comprises of a plurality of rows 116, each row 116 extending about a perimeter (e.g., circumference) of the access cannula 102 and including a slot 118. Each row 116 is separated from an adjacent one of the rows 116 by a desired distance along a length of the access cannula 102. Each slot 118 extends along a corresponding one of the rows 116 over a selected portion of the circumference of the distal portion 108 and extends through a wall 120 defining the access cannula 102 so that the channel 114 is open to and in communication with an exterior of the access cannula 102 via the slots 118. In an exemplary embodiment, the rows 116 are equidistantly spaced from one another along a length of the distal portion 108. It will be understood by those of skill in the art, however, that depending on the laser cut pattern 106, the rows 116 may not be equidistantly spaced and, in one embodiment, the spacing between the rows 116 alternates between 2 or more different distances.

In an exemplary embodiment, the rows 116 within which the slots 118 extend are substantially perpendicularly to the longitudinal axis L of the cannula 102. In one embodiment, each of the slots 118 corresponds in length (e.g., an extent of the slot along the axis L) and width (e.g., a distance around a circumference of the distal portion 108 along which the slot 118 extends). It will be understood by those of skill in the art, however, that the access cannula 102 does not require each of the slots 118 to have the same dimensions and that, in another embodiment, the slots 118 may have variable dimensions to from a pattern 106 achieving a desired level of compression. In an exemplary embodiment, each of the rows 116 includes a plurality of slots 118 within rows equidistantly spaced from one another. However, it will be understood by those of skill in the art that, depending on the pattern 106, any of the rows 116 may have one or more slots 118 with variable spacing therebetween.

As described above, the laser cut pattern 106 may be selected based on a desired compressibility of the access cannula 102. The laser cut pattern 106 determines a compressibility based on factors such as, for example, a number of slots 118 per row 116, a width and length of each slot 118, a rotation angle between slots 118 in adjacent rows 116, and a distance between rows 116. Each variable has its own effect on the compressibility of the access cannula 102. The dimensions of the slots 118 (e.g., width, length) determine how much each row will compress. For example, a wide, long slot 118 will remove more material from the wall 120 of the access cannula 102 than a thin, short slot 118 to allow for greater compressibility. In particular, a wider, longer slot 118 leaves more room for portions of the access cannula 102 extending between the rows 116 to bend, thereby reducing a length of the access cannula 102 as the access cannula 102 is longitudinally compressed.

The rotation angle of slots 118 in adjacent rows 116 may also be adjusted to attain a desired compression of the access cannula 102 since, as will be understood by those of skill in the art, the rotation angle will affect the flexibility of the access cannula 102. The rotation angle may be defined as a rotation of a slot 118 in a first one of the rows 116 about the longitudinal axis L relative to a corresponding slot 118 in a second, adjacent one of the rows 116. For example, when the slots 118 in the adjacent rows 116 have a rotation angle of 90 degrees and the slots 118 have equal dimensions, the slot 118 in the first row 116 will be offset from the slot 118 in the second row by an angle of 90 degrees about the longitudinal axis of the access cannula 102. In other words, uncut sections 122 of the access cannula 102 between adjacent slots 118 alternate at right angles with each adjacent row 116.

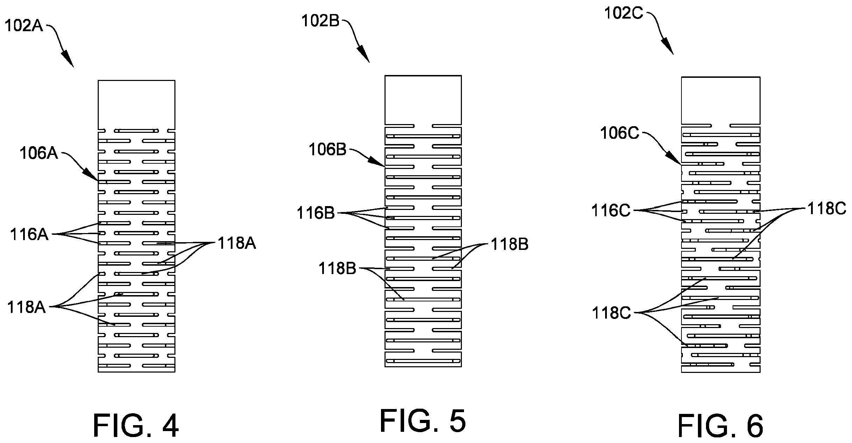

Figs. 4-6 show examples of patterns 106 (e.g., laser cut patterns) which may be used to achieve varying levels of compressibility. Fig. 4 shows a distal portion of an access cannula 102A including a laser cut pattern 106A in which each row 116A of the pattern 106A includes four equal slots 118A, a rotation angle of approximately 45 degrees between adjacent rows 116A. Fig. 5 shows a distal portion of an access cannula 102B according to another exemplary embodiment. A laser cut pattern 106B along the access cannula 102B, each row 116B of the pattern 106B including two slots 118B with a rotation angle of 90 degrees between adjacent rows 116B. As shown, the slots 118B of the laser cut pattern 106B are longer than the slots 118A of the laser cut pattern 106A as shown in Fig. 3. Thus, it will be understood by those of skill in art that the access cannula 102B will have a greater level of longitudinal compression than the access cannula 102A when subject to the same compressive force. In addition, the laser cut pattern 106B, with the 90-degree rotation angle, maximizes both a compressibility and flexibility of the distal portion 108.

Fig. 6 shows a distal portion 108C of an access cannula 102C according to another example. The access cannula 102C includes a pattern 106C in which each row 116C includes two slots 118C, similarly to the access cannula 102B, shown in Fig. 4. Rather than a rotation angle of 90 degrees between adjacent rows 116C, however, the laser cut pattern 106C includes a rotation angle of 85 degrees between adjacent rows 116C. Thus, the laser cut pattern 106C reduces the longitudinal flexibility and compressibility of the access cannula 102C relative to the access cannula 102B. It will be understood by those of skill in the art, however, that the rotation angle between rows may be selected and/or varied to separately tune the flexibility and compressibility of the access cannula 102, as desired.

It will be understood by those of skill in the art that the patterns 106A, 106B, and 106C shown in Figs. 4-6, respectively, are exemplary only and that the pattern 106 of the system 100 may take any of a variety of configurations to achieve a desired level of compressibility and/or flexibility. In particular, the pattern facilitates movement of the access cannula 102 between the non-compressed configuration and the compressed configuration, during which the access cannula 102 is axially compressed along the longitudinal axis L via a desired distance. As exemplified by the embodiments described and shown in Figs. 4-6, a desired level of compressibility and/or flexibility may be achieved by changing features such as, for example, a number of slots 118 per row 116, a width and length of each slot 118, a rotation angle between slots 118 in adjacent rows 116, and a distance between rows 116 of the laser cut pattern 106.

In an exemplary embodiment, the access cannula 102 is sized, shaped and configured to be inserted through, for example, a working channel of an endoscope and should, in some cases, therefore have sufficient flexibility to be inserted through even tortuous paths of a body lumen that are traversed by flexible endoscopes. In one embodiment, the access cannula 102 is formed of a nitinol hypotube, which has superelastic properties so that the access cannula 102 is configured to be moved from the non-compressed to the compressed configuration when subject to a compressive force exerted thereon and, upon release of the compressive force, will revert towards the non-compressed configuration. Along with the pattern 106, properties of the access cannula 102 may be further fine-tuned by controlling how the Nitinol is processed.

As will be understood by those of skill in the art, when Nitinol experiences a strain, a phase change from Austenite to Martensite can be induced within the material, giving it its superelasticity. The temperature at which the Nitinol is fully Austenitic can be controlled via heat treatment of the material to determine a desired behavior (e.g., stiffness or flexibility) of the material at that temperature (e.g., body temperature). While Nitinol may be used to further fine tune desired properties of the access cannula 102, it will be understood by those of skill in the art, however, that the access cannula 102 may be formed of any of a variety of materials so long as the access cannula 102 is configured to be movable between the non-compressed and the compressed configurations, as will be described in herein.

According to another exemplary embodiment, a thin, flexible coating, jacket, sheath or other covering may extend over at least the distal portion 108 along which the pattern 106 extends to increase a stiffness thereof and/or better control a compressibility thereof based on a desired application of the system 100. In this embodiment, the coating, jacket, sheath or other covering would allow compression of the access cannula 102 while preventing fluid communication between the channel 114 thereof and an exterior of the access cannula 102 via the slots 118. In another exemplary embodiment, the coating, jacket, sheath or other covering may be formed of multiple durometer materials to induce variable stiffness along desired portions of the access cannula 102. This jacket or covering, along with the pattern 106 may be used to achieve desired characteristics of the access cannula.

In addition, while the exemplary embodiments describe patterns 106 being used to achieve a desired compressibility and/or flexibility of the access cannula 102, the pattern 106 may also be selected to achieve a desired echogenicity of the access cannula 102. In particular, the slots 118 may be formed through the wall 120 to create surface features which increase echogenicity and optimize visualization under ultrasound guidance so that a user may be confident that the access cannula 102 is being positioned against a desired portion of a wall of the organ and/or target duct. It will be understood by those of skill in the art, however, that the access cannula 102 may include other surface features in addition to or in place of the pattern 106 to facilitate visualization under ultrasound guidance.

The needle 104 extends longitudinally from a proximal end to the sharp distal end 110 and is sized, shaped, and configured to be received within the channel 114. The sharp end 110 tapers toward a distal tip 124. A length of the of the needle 104 may correspond to the length of the access cannula 102 so that, when the distal portion 108 is in the non-compressed configuration, the needle 104 is substantially housed within the channel 114 of the cannula 102. In an exemplary embodiment, proximal ends of the needle 104 and the access cannula 102 are fixed relative to one another so that, in the non-compressed configuration, only a distal tip 124 of the sharp end 110 extends distally past the distal end 112 of the access cannula 102. Thus, when the access cannula is initially positioned against a wall of an organ/duct, the distal tip 124 engages the wall so that the access cannula 102 is prevented from moving as it is pressed against the wall to apply a compressive force thereto. In other words, the exposed distal tip 124 prevents the system 100 from sliding along the organ/duct wall to ensure that the user stays on target.

With the sharp distal tip 124 partially exposed, the distal tip 124 will catch the tissue, begin to puncture, and once the tissue wall comes into contact with the distal end 112 of the access cannula 102, the access cannula 102 will begin to compress while the sharp end 110 continues its trajectory into the tissue. In another embodiment, however, the sharp end 110 may be entirely housed within the channel 114 when the access cannula 102 is in the non-compressed configuration. The length of the needle 104 is selected so that, upon compression of the access cannula 102, a desired length of the sharp end 110 is exposed to puncture the wall without puncturing, for example, a far wall of the target duct as has been described above.

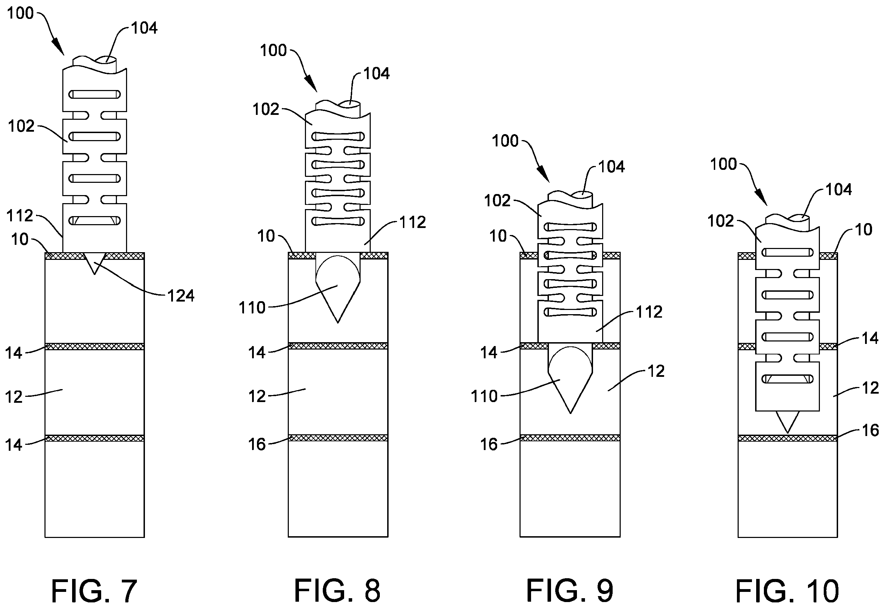

According to an exemplary method, as shown in Figs. 7-10, for treating a biliary duct using the system 100, an insertion device such as, for example, an endoscope is inserted through a body lumen until a distal end thereof is positioned in a target area of a patient body (e.g., within a stomach of a patient, proximate a target duct to be accessed). When the endoscope has been positioned as desired, the system 100, in the non-compressed configuration, is inserted through a working channel of the endoscope until the distal end 112 of the access cannula 102 extends out of the working channel to contact a desired site on the stomach wall 10 (e.g., along a portion of the stomach wall 10 corresponding to an area of the target duct 12 to be accessed), as shown in Fig. 7. As described above, in an exemplary embodiment, the distal tip 124 of the sharp end 110 of the needle 104 protrudes slightly from the distal end 112 of the access cannula 102 when the distal portion 108 is in the non-compressed configuration so that the distal tip 124 engages the desired site on the stomach wall 10 and the position of the access cannula 102 is secured.

As shown in Fig 8, the access cannula 102 and needle 104 are then pressed distally against the stomach wall 10 until the access cannula 102 moves from the non-compressed configuration toward the compressed configuration, exposing the sharp end 110 which punctures the stomach wall 10. As described above, as the access cannula 102 is moved toward the compressed configuration, the access cannula 102 is axially compressed, along the longitudinal axis L, shortening the access cannula 102 and allowing the sharp end 110 of the needle 104 to move distally relative to the endoscope to puncture the wall 10 of the stomach.

When the stomach wall 10 is punctured, the restraining force exerted against the distal end of the distal portion 108 is reduced or equalized to the compressive force of the access cannula 102 and the access cannula 102 follows the sharp end 110 distally through the stomach wall 10. The pattern 106 is designed to only compress a certain amount before the distal pushing force overpowers the force applied via the stomach wall 10. The user may then continue to move the cannula 102 and the needle 104 distally until the sharp end 110 of the needle 104 punctures a near wall 14 of the target duct, as shown in Fig. 9. As shown in Fig. 8 and 9, the sharp end 110 remains exposed while the distal portion 108 of the access cannula 102 passes through the stomach wall 10 and any other intervening tissue between the stomach wall 10 and the duct wall 14 due to the resistance (compressive force) exerted on the distal portion 108 by this tissue. As would be understood by those skilled in the art, this compressive force will depend on factors such as the stiffness, rigidity and/or texture of this tissue.

As discussed above, a compressibility determined via the pattern 106 along the distal portion 108 of the access cannula 102 and a length of the pattern 106 along the distal portion 108 of the access cannula 102 and/or a length of the needle 104 is selected based on factors such as, for example, a diameter of the target duct 12 so that, when the exposed sharp end 110 enters the target duct 12 and before the sharp end pierces a far wall 16 of the target duct 12, the distal end of the distal portion 108 will enter the target duct 12 and the distal portion 108 will revert toward the non-compressed configuration (i.e., when the resistance applied to the distal portion 108 drops as the distal portion 108 enters the lumen of the duct 12).

According to an exemplary embodiment, a length of the compression of the distal portion 108 is selected so that, when the sharp end 110 penetrates the duct wall 14 and enters the duct 12, the distal end of the distal portion 108 has entered the duct 12 and expanded over the needle 104 before the sharp end 110 can pass through the far wall 16 of the target duct 12. The pattern 106 and the material of which the access cannula 102 is formed are configured so that, once the sharp end 110 has pierced the near duct wall 14, the access cannula 102 reverts toward the non-compressed configuration, extending distally over the sharp end 110 and into the target duct 12, as shown in Fig. 10.

Once the target duct has been accessed via the access cannula 102, the needle 104 may be removed therefrom, leaving the access cannula 102 in place. It will be understood by those of skill in the art that upon piercing of the target duct, reversion of the access cannula toward the non-compressed configuration increases a length of the access cannula within the target duct, thereby securing the access cannula therein for the passage of other devices therethrough. In an exemplary embodiment, for example, a guidewire may be passed through the access cannula 102 into the target duct 12 so that a catheter or other device may be inserted thereover to facilitate drainage of the target duct. The method described above is an exemplary method for facilitating a draining of target duct. It will be understood by those of skill in the art, however, that the system 100 may be utilized for other applications in which a longitudinally compressible access cannula 102 may be desired.

It will be appreciated by those skilled in the art that changes may be made to the embodiments described above without departing from the inventive concept thereof. It should further be appreciated that structural features and methods associated with one of the embodiments can be incorporated into other embodiments. It is understood, therefore, that this invention is not limited to the particular embodiment disclosed, but rather modifications are also covered within the scope of the present invention as defined by the appended claims.

## Claims

1. A system for accessing a biliary duct, comprising:
an access cannula (102) extending along a longitudinal axis (L) from a proximal end to a distal end (112) and including a channel (114) extending longitudinally therethrough, a distal portion (108) of the access cannula (102) including a laser cut pattern (106; 106A; 106B; 106C) including a plurality of slots (118; 118A; 118B; 118C) extending through a wall (120) thereof so that the access cannula (102) is movable between a biased non-compressed configuration and a compressed configuration, in which the access cannula (102) is compressed along the longitudinal axis (L); and
a needle (104) extending longitudinally from a proximal end to a sharp distal end (110), the needle (104) housed within the channel (114) of the access cannula (102) so that, when the access cannula (102) is in the non-compressed configuration, the sharp distal end (110) is covered via the access cannula (102) and, when the access cannula (102) is in the compressed configuration, the sharp distal end (110) is exposed to puncture a wall (10, 14, 16) of one of an organ and a target duct (12).

2. The system of claim 1, wherein the laser cut pattern (106; 106A; 106B; 106C) includes a plurality of rows (116; 116A; 116B; 116C), each row (116; 116A; 116B; 116C) extending about a periphery of the access cannula (102), each of the rows (116; 116A; 116B; 116C) including at least one slot (118; 118A; 118B; 118C) extending along a portion of the row, through the wall (120) such that the channel (114) of the access cannula (102) is open to and in communication with an exterior of the access cannula (102) via the slots (118; 118A; 118B; 118C).

3. The system of claim 2, wherein each row (116; 116A; 116B; 116C) of the laser cut pattern (106; 106A; 106B; 106C) extends perpendicular to the longitudinal axis (L) of the access cannula (102).

4. The system of any one of claims 1-3, wherein the laser cut pattern (106; 106A; 106B; 106C) is selected to achieve a desired level of compressibility for the access cannula (102), the desired level of compressibility including a compressive force required to move the access cannula (102) from the non-compressed configuration toward the compressed configuration and length by which the access cannula (102) is shortened when moved from the non-compressed configuration toward the compressed configuration.

5. The system of claim 4, wherein the desired level of compressibility achieved via the laser cut pattern (106; 106A; 106B; 106C) is based on one of a number of slots (118; 118A; 118B; 118C) per row (116; 116A; 116B; 116C), dimensions of each of the slots (118; 118A; 118B; 118C), a rotation angle between corresponding slots (118; 118A; 118B; 118C) in adjacent rows (116; 116A; 116B; 116C), and a distance between rows (116; 116A; 116B; 116C).

6. The system of any one of claims 1-5, wherein, in the non-compressed configuration, a distal-most tip (124) of the needle (104) protrudes distally of the distal end (112) of the access cannula (102).

7. The system of any one of claims 1-6, wherein, when the access cannula (102) is moved from the non-compressed configuration to the compressed configuration, the access cannula (102) is shortened via a distance corresponding to the sharp distal end (110) of the needle (104).

8. The system of claim 1, wherein a length of the sharp distal end (110) of the needle (104) that is exposed when the access cannula (102) is in the compressed configuration is smaller than the target duct (12) such to prevent a through and through puncture.

9. The system of any one of claims 1-8, wherein the access cannula (102) is formed of a nitinol hypotube.

10. The system of any of claims 1-9, wherein
the access cannula (102) has a substantially tubular body and is sized, shaped and configured to be inserted through a working channel of an endoscope; and
the needle (104) being removably received within the access cannula (102).

## Patentansprüche

1. System für Zugang zu einem Gallengang, aufweisend:
eine Zugangskanüle (102), die sich entlang einer Längsachse (L) von einem proximalen Ende zu einem distalen Ende (112) erstreckt und einen Kanal (114) aufweist, der sich in Längsrichtung durch die Kanüle erstreckt, wobei ein distaler Abschnitt (108) der Zugangskanüle (102) ein Laserschnittmuster (106; 106A; 106B; 106C) mit mehreren sich durch eine Wand (120) der Kanüle erstreckenden Schlitzen (118; 118A; 118B; 118C) aufweist, so dass die Zugangskanüle (102) bewegbar ist zwischen einer vorgespannten nichtkomprimierten Konfiguration und einer komprimierten Konfiguration, in der die Zugangskanüle (102) entlang der Längsachse (L) komprimiert ist; und
eine Nadel (104), die sich in Längsrichtung von einem proximalen Ende zu einem scharfen distalen Ende (110) erstreckt, wobei die Nadel (104) in dem Kanal (114) der Zugangskanüle (102) untergebracht ist, so dass, wenn die Zugangskanüle (102) in der nichtkomprimierten Konfiguration ist, das scharfe distale Ende (110) durch die Zugangskanüle (102) verdeckt ist, und wenn die Zugangskanüle (102) in der komprimierten Konfiguration ist, das scharfe distale Ende (110) freiliegt, um eine Wand (10, 14, 16) eines Organs oder eines Zielganges (12) zu punktieren.

2. System nach Anspruch 1, wobei das Laserschnittmuster (106; 106A; 106B; 106C) mehrere Reihen (116; 116A; 116B; 116C) aufweist, wobei jede Reihe (116; 116A; 116B; 116C) sich um einen Umfang der Zugangskanüle (102) erstreckt, jede der Reihen (116; 116A; 116B; 116C) mindestens einen Schlitz (118; 118A; 118B; 118C) aufweist, der sich entlang eines Teils der Reihe durch die Wand (120) erstreckt, so dass der Kanal (114) der Zugangskanüle (102) durch die Schlitze (118; 118A; 118B; 118C) offen zu und in Verbindung mit einer Außenumgebung der Zugangskanüle (102) ist.

3. System nach Anspruch 2, wobei jede Reihe (116; 116A; 116B; 116C) des Laserschnittmusters (106; 106A; 106B; 106C) sich senkrecht zu der Längsachse (L) der Zugangskanüle (102) erstreckt.

4. System nach einem der Ansprüche 1 bis 3, wobei das Laserschnittmuster (106; 106A; 106B; 106C) gewählt ist, um ein gewünschtes Maß an Komprimierbarkeit der Zugangskanüle (102) zu erzielen, wobei das gewünschte Maß an Komprimierbarkeit umfasst: eine zum Bewegen der Zugangskanüle (102) aus der nichtkomprimierten Konfiguration hin zu der komprimierten Konfiguration erforderliche Kompressionskraft und eine Länge, um die die Zugangskanüle (102) verkürzt wird, wenn sie aus der nichtkomprimierten Konfiguration hin zu der komprimierten Konfiguration bewegt wird.

5. System nach Anspruch 4, wobei das gewünschte Maß an Komprimierbarkeit, das durch das Laserschnittmuster (106; 106A; 106B; 106C) erzielt wird, auf einer Anzahl von Schlitzen (118; 118A; 118B; 118C) pro Reihe (116; 116A; 116B; 116C) oder Abmessungen jedes der Schlitze (118; 118A; 118B; 118C) oder einem Rotationswinkel zwischen entsprechenden Schlitzen (118; 118A; 118B; 118C) in benachbarten Reihen (116; 116A; 116B; 116C) oder einer Distanz zwischen Reihen (116; 116A; 116B; 116C) basiert.

6. System nach einem der Ansprüche 1 bis 5, wobei in der nichtkomprimierten Konfiguration eine äußerste distale Spitze (124) der Nadel (104) distal des distalen Endes (112) der Zugangskanüle (102) vorragt.

7. System nach einem der Ansprüche 1 bis 6, wobei, wenn die Zugangskanüle (102) aus der nichtkomprimierten Konfiguration in die komprimierte Konfiguration bewegt wird, die Zugangskanüle (102) um eine Distanz verkürzt wird, die dem scharfen distalen Ende (110) der Nadel (104) entspricht.

8. System nach Anspruch 1, wobei eine in der komprimierten Konfiguration der Zugangskanüle (102) freiliegende Länge des scharfen distalen Endes (110) der Nadel (104) kleiner als der Zielgang (12) ist, um so einen Durchstich zu verhindern.

9. System nach einem der Ansprüche 1 bis 8, wobei die Zugangskanüle (102) aus einem Nitinol-Hyporöhrchen besteht.

10. System nach einem der Ansprüche 1 bis 9, wobei
die Zugangskanüle (102) einen im Wesentlichen röhrenförmigen Körper hat und bemessen, gestaltet und konfiguriert ist, um durch einen Arbeitskanal eines Endoskops eingeführt zu werden; und
die Nadel (104) entnehmbar in der Zugangskanüle (102) aufgenommen ist.

## Revendications

1. Système pour accéder à un canal biliaire, comprenant :
une canule d'accès (102) s'étendant le long d'un axe longitudinal (L) d'une extrémité proximale à une extrémité distale (112) et comprenant un canal (114) s'étendant de manière longitudinale à travers cette dernière, une partie distale (108) de la canule d'accès (102) comprenant un motif découpé au laser (106 ; 106A ; 106B ; 106C) comprenant une pluralité de fentes (118 ; 118A ; 118B ; 118C) s'étendant à travers sa paroi (120) de sorte que la canule d'accès (102) est mobile entre une configuration non comprimée sollicitée et une configuration comprimée, dans laquelle la canule d'accès (102) est comprimée le long de l'axe longitudinal (L) ; et
une aiguille (104) s'étendant longitudinalement à partir d'une extrémité proximale jusqu'à une extrémité distale pointue (110), l'aiguille (104) étant logée à l'intérieur du canal (114) de la canule d'accès (102) de sorte que, lorsque la canule d'accès (102) est dans la configuration non comprimée, l'extrémité distale pointue (110) est recouverte via la canule d'accès (102) et, lorsque la canule d'accès (102) est dans la configuration comprimée, l'extrémité distale pointue (110) est exposée pour piquer une paroi (10, 14, 16) de l'un parmi un organe et un conduit cible (12).

2. Système selon la revendication 1, dans lequel le motif découpé au laser (106 ; 106A ; 106B ; 106C) comprend une pluralité de rangées (116 ; 116A ; 116B ; 116C), chaque rangée (116 ; 116A ; 116B, 116C) s'étendant autour d'une périphérie de la canule d'accès (102), chacune des rangées (116 ; 116A ; 116B ; 116C) comprenant au moins une fente (118 ; 118A ; 118B ; 118C) s'étendant le long d'une partie de la rangée, à travers la paroi (120) de sorte que le canal (114) de la canule d'accès (102) est ouvert sur et est en communication avec un extérieur de la canule d'accès (102) via les fentes (118 ; 118A ; 118B ; 118C).

3. Système selon la revendication 2, dans lequel chaque rangée (116 ; 116A ; 116B ; 116C) du motif découpé au laser (106 ; 106A ; 106B ; 106C) s'étend perpendiculairement à l'axe longitudinal (L) de la canule d'accès (102).

4. Système selon l'une quelconque des revendications 1 à 3, dans lequel le motif découpé au laser (106 ; 106A ; 106B ; 106C) est sélectionné pour obtenir un niveau de compressibilité souhaité pour la canule d'accès (102), le niveau de compressibilité souhaité comprenant une force de compression nécessaire pour déplacer la canule d'accès (102) de la configuration non comprimée vers la configuration comprimée et la longueur grâce à laquelle la canule d'accès (102) est raccourcie lorsqu'elle est déplacée de la configuration non comprimée vers la configuration comprimée.

5. Système selon la revendication 4, dans lequel le niveau de compressibilité souhaité obtenu via le motif découpé au laser (106 ; 106A ; 106B ; 106C) est basé sur l'un parmi un certain nombre de fentes (118 ; 118A ; 118B ; 118C) par rangée (116 ; 116A ; 116B ; 116C), les dimensions de chacune des fentes (118 ; 118A : 118B ; 118C), un angle de rotation entre les fentes (118 ; 118A ; 118B ; 118C) correspondantes dans les rangées (116 ; 116A ; 116B ; 116C) adjacentes et une distance entre les rangées (116 ; 116A ; 116B ; 116C).

6. Système selon l'une quelconque des revendications 1 à 5, dans lequel, dans la configuration non comprimée, la pointe la plus distale (124) de l'aiguille (104) fait saillie de manière distale par rapport à l'extrémité distale (112) de la canule d'accès (102).

7. Système selon l'une quelconque des revendications 1 à 6, dans lequel, lorsque la canule d'accès (102) est déplacée de la configuration non comprimée à la configuration comprimée, la canule d'accès (102) est raccourcie via une distance correspondant à l'extrémité distale pointue (110) de l'aiguille (104).

8. Système selon la revendication 1, dans lequel une longueur de l'extrémité distale pointue (110) de l'aiguille (104) qui est exposée lorsque la canule d'accès (102) est dans la configuration comprimée, est inférieure au conduit cible (12) afin d'empêcher une ponction transfixiante.

9. Système selon l'une quelconque des revendications 1 à 8, dans lequel la canule d'accès (102) est formée avec un hypotube en Nitinol.

10. Système selon l'une quelconque des revendications 1 à 9, dans lequel :
la canule d'accès (102) a un corps sensiblement tubulaire et est dimensionnée, formée et configurée pour être insérée dans un canal de travail d'un endoscope ; et
l'aiguille (104) étant reçue, de manière amovible, à l'intérieur de la canule d'accès (102).
